# EUROPÄISCHE PATENTSCHRIFT

(11) **EP 1 239 958 B1**
(45) Veröffentlichungstag und Bekanntmachung des Hinweises auf die Patenterteilung: **29.09.2004**
(21) Anmeldenummer: 00979574.1
(22) Anmeldetag: 16.11.2000
(51) Int. Cl.: B01J 31/08, B01J 37/08, C07C 37/20

(54) **VERFAHREN ZUR KONDITIONIERUNG VON IONENAUSTAUSCHERN**
PROCESS FOR CONDITIONING ION EXCHANGE RESINS
PROCEDE POUR CONDITIONNER DES RESINES ECHANGEUSES D'IONS

(30) Priorität: 23.11.1999 DE 19956229; 06.06.2000 DE 10027908
(43) Veröffentlichungstag der Anmeldung: 18.09.2002
(73) Patentinhaber: Bayer MaterialScience AG, 51368 Leverkusen (DE)
(72) Erfinder: BÖDIGER, Michael, League City, TX 77573 (US); NEUMANN, Rainer, 47803 Krefeld (DE); HEYDENREICH, Frieder, 40593 Düsseldorf (DE); PREIN, Michael, B-2930 Brasschaat (BE); FENNHOFF, Gerhard, 47877 Willich (DE); SCHNEGG, Ulrich, 51377 Leverkusen (DE); WAGNER, Rudolf, 51061 Köln (DE); WAMBACH, Wolfgang, 51065 Köln (DE); KLIPPER, Reinhold, 50933 Köln (DE)
(86) Internationale Anmeldenummer: PCT/EP2000/011352
(87) Internationale Veröffentlichungsnummer: WO 2001/037992

(56) Entgegenhaltungen:
- EP-A- 0 765 685
- US-A- 5 146 007
- US-A- 5 502 016

## Beschreibung

Die Anmeldung betrifft die Konditionierung und Verwendung von Ionenaustauschern für die Herstellung von Bisphenolen.

Die vorliegende Anmeldung betrifft insbesondere die Konditionierung von monodispersen Kationen- und Anionenaustauschern sowie deren Verwendung als Katalysatoren, insbesondere zur Katalyse von Kondensationsreaktionen.

Kondensationsreaktionen sind literaturbekannt, wie z. B. die Synthese von Bisphenolen, die im allgemeinen durch säurekatalysierte Umsetzung von Phenolen mit Carbonylverbindungen erfolgt. Letztere Reaktion wird im allgemeinen in Festbettoder Wirbelbettreaktoren wie auch in Reaktivkolonnen durchgeführt.

Beispielsweise werden bei der Synthese von Bisphenolen üblicherweise Katalysatoren aus vernetzten sulfonierten Polystyrolharzen (saure Ionentauscher) zum Einsatz gebracht. Diese Ionenaustauscher können gegebenenfalls durch kovalent- oder ionisch gebundene Cokatalysatoren chemisch modifiziert sein und sind makroporös oder gelförmig (US-A-4,191, 843; US-A-3,037,052). Gemäß US-A-5,233,096 werden für die Kondensationsreaktion des Phenols mit Aldehyden oder Ketonen zu Bisphenolen gejettete, suspensionspolymerisierte Styrol-Copolymere, die mit stark sauren funktionellen Gruppen funktionalisiert wurden, eingesetzt.

In der Handelsform enthalten die Ionentauscher 45 bis 85 Gew.-% Wasser. Um eine Konkurrenz von Wasser zu den Einsatzstoffen an den katalytischen Stellen und somit eine Verringerung der Aktivität des Ionentauschers zu vermeiden, sollte das Wasser, zum Beispiel für den Einsatz bei der Herstellung des technisch wichtigen 2,2-Bis(4-hydroxyphenyl)propans (BPA), weitgehend entfernt werden. Darüber hinaus sind handelsübliche Ionentauscher aufgrund ihres Herstellverfahrens mit gewissen Mengen an sauren Oligomeranteilen versehen, die bei kontinuierlichem Durchströmen mit Reaktionslösung ausgewaschen werden können und die Reinheit, Thermostabilität und Farbe von hiermit hergestellten Produkten nachteilig beeinflussen.

Zur Verbesserung der Raum-Zeit-Ausbeuten und zur Erhöhung der Selektivitäten von Herstellungsverfahren in denen diese Ionenaustauscher als Katalysatoren eingesetzt ist es deshalb nötig, den Katalysator vor dem ersten Einsatz zu konditionieren.

Aufgabe der vorliegenden Erfindung ist daher, Ionenaustauscher auf Basis von vernetzten sulfonierten Polystyrolharzen so zu konditionieren, dass eine Kontamination des Reaktionsprodukts mit sauren Bruchstücken bei Kondensationsreaktionen verhindert wird. Im Falle der bereits oben beschriebenen Synthese von Bisphenolen soll somit ein problemloses Anfahren mit Phenol/Aceton-Mischungen gewährleistet werden, ohne dass Aktivierungsverluste bei der Überführung des so konditionierten Ionentauschers in das Reaktionsgefäß auftreten und so die bei der Konditionierung anfallenden Stoffströme sinnvoll aufgearbeitet und gegebenenfalls zurückgeführt werden können, ohne dass unnötiger Materialverlust auftritt.

Im Falle der Synthese von Bisphenolen sind in der Literatur zur Lösung dieser Aufgabe verschiedene Ansätze beschrieben worden. So kann ein wasserhaltiger Ionentauscher im Reaktionsgefäß durch Spülen mit Phenol entwässert werden, wobei Wasser von durchströmendem Phenol mitgenommen wird. Hierbei tritt jedoch eine Schrumpfung des Ionentauschers ein, die zu einer zusätzlichen mechanischen Belastung des Ionenaustauschers führt und somit Bruch der Körner zur Folge haben kann. Außerdem werden Oligomerspuren bei diesem Vorgehen nur unzureichend entfernt. Darüber hinaus ist dieser Prozess zeitaufwendig und das Reaktionsgefäß steht in diesem Zeitraum nicht für die Produktion zur Verfügung.

In US-A-3,037,052 wird daher empfohlen, den Ionenaustauscher vor dem Gebrauch zur Synthese von Bisphenolen bei erhöhten Temperaturen (ca. 150°C) zu trocknen. Hierbei muss jedoch die Trockengeschwindigkeit sehr genau kontrolliert werden (vgl. Zundel et al. Physik. Chem. (Frankfurt), 59, 225 (1968)) und es besteht die Gefahr einer mechanischen Schädigung bei den hohen Temperaturen.

In US-A-5,146,007 ist die teilweise Entwässerung des Katalysators vor dem Umfüllen in den Reaktor beschrieben. 20 bis 90 % des Wassers werden durch Vakuumtrocknung oder Trocknung mit einem Schleppgasstrom entfernt. Anschließend wird der Katalysator in den Reaktor überführt und das Restwasser durch Spülen mit Phenol entfernt. Bei einem derartigen Vorgehen werden jedoch schädliche wassserlösliche Oligomeranteile nicht ausreichend entfernt.

In EP-A-765 685 wird vorgeschlagen, den Katalysator zunächst mit Wasser bis zu einer definierten Restleitfähigkeit zu waschen, anschließend einen Teil des Wassers durch Vakuum- oder Schleppgastrocknung zu entfernen und anschließend durch eine kontinuierliche Spülung mit Phenol den Wassergehalt auf bis zu <1 % zu reduzieren. Während dieses Vorgehen für eine Abtrennung von Oligomerbestandteilen sorgt, ist es in der praktischen Durchführung umständlich, da spezielle Vorrichtungen zur teilweisen Wasserentfernung durch Vakuum- oder Schleppgastechnik nötig sind. Darüber hinaus fallen große Mengen Wasser und Phenol an, die mit schädlichen Katalysatorbestandteilen kontaminiert sind und nicht ohne weiteres in den Prozess rückgeführt werden können. Bei Durchführung von Wasserwäsche und Entwässerung im Reaktorbehälter entsteht Produktionsausfall durch die Blockierung des Behälters für die genannten Konditionierungsarbeiten.

Um die oben beschriebenen Nachteile der verschiedenen Konditionierungsverfahren zu umgehen, wird ein integriertes Verfahren zur Katalysatorpräparation entwickelt, das für die Konditionierung von Ionenaustauschern, die in Kondensationsreaktionen eingesetzt werden sollen, geeignet ist.

Gegenstand der Anmeldung ist daher ein Verfahren zur Konditionierung von Ionenaustauschem, das dadurch gekennzeichnet ist, dass
a) der wasserfeuchte Ionentauscher in einer Einheit (1) mit sauerstoffreiem VE-Wasser bei 5 bis 80°C, insbesondere 20 bis 60°C suspendiert wird; wobei insbesondere ein Volumenverhältnis von 3 bis 1,5 Teilen, bevorzugt 2,5 bis 2 Teilen wasserfeuchtem Ionentauscher zu 1 Teil Wasser eingestellt wird und wobei der Gehalt an gelöstem Sauerstoff im eingesetzten VE-Wassers vorzugsweise nicht grösser als 1 ppm, bevorzugt nicht grösser als 100 ppb, ist, und wobei der Gehalt an gelösten oder ungelösten Metallionen im verwendeten VE-Wasser vorzugsweise nicht grösser als 1 ppm, bevorzugt nicht grösser als 0,5 ppm für Fe, Co, Ni, Mo, Cr, Cu als individuelle Komponenten ist und vorzugsweise nicht größer als 10 ppm, bevorzugt nicht größer als 1 ppm für die Summe der genannten Metalle ist;
b) die Suspension, vorzugsweise durch ½ bis 24-stündiges Rühren bei 5 bis 80°C, insbesondere 20 bis 60°C bewegt wird und anschließend eine Analyse der überstehenden wässrigen Lösung auf ihre Leitfähigkeit durchgeführt wird, um einen Anhaltspunkt über den Oligomerengehalt des Ionentauschers und die Anzahl der nötigen Waschzyklen für Schritt c) zu erhalten;
c) der Ionenaustauscher einer diskontinuierlichen Wäsche mit sauerstofffreiem VE-Wasser bis zu konstanter Restleitfähigkeit unterzogen wird, in Abhängigkeit der Leitfähigkeit wird vorzugsweise 5 bis 50, insbesondere 10 bis 25 mal gewaschen; wobei nach Ablassen des VE-Wassers jeweils 0,2 bis 1,5 Volumenteile VE-Wasser, bezogen auf den wasserfeuchten Ionentauscher hinzugefügt, die Mischung bewegt und das VE-Wasser abgelassen und am Ablauf die Leitfähigkeit des VE-Wassers überprüft wird, wobei die Leitfähigkeit am Ende der Waschzyklen kleiner 100 mikro-Siemens/cm, bevorzugt kleiner 50 mikro-Siemens/cm, besonders bevorzugt kleiner 20 mikro-Siemens/cm ist, und wobei der Gehalt an gelöstem Sauerstoff im eingesetzten VE-Wasser vorzugsweise nicht grösser als 1 ppm, bevorzugt nicht grösser als 100 ppb, ist und wobei der Gehalt an gelösten oder ungelösten Metallionen im verwendeten VE-Wasser vorzugsweise nicht größer als 1 ppm, bevorzugt nicht größer als 0,5 ppm für Fe, Co, Ni, Mo, Cr, Cu als individuelle Komponenten ist und vorzugsweise nicht größer als 10 ppm, bevorzugt nicht grösser als 1 ppm für die Summe der genannten Metalle ist;
d) nach der letzten Wäsche das VE-Wasser abgelassen und dem Ionenaustauscher sauerstofffreies Phenol bei 50 bis 90°C, insbesondere 60 bis 80°C zugemischt wird; wobei vorzugsweise ein Volumenverhältnis von 0,60 bis 1,5 Teilen von wasserfeuchtem Ionenaustauscher zu einem Teil Phenol eingestellt wird und wobei vorzugsweise der Gehalt an gelöstem Sauerstoff des verwendeten Phenols kleiner 1 ppm, bevorzugt kleiner 100 ppb ist, und wobei der Gehalt an gelösten oder ungelösten Metallionen im verwendeten Phenol vorzugsweise nicht grösser als 1 ppm, bevorzugt nicht grösser als 0,5 ppm für Fe, Co, Ni, Mo, Cr, Cu als individuelle Komponenten ist und vorzugsweise nicht größer als 10 ppm, bevorzugt nicht grösser als 1 ppm für die Summe der genannten Metalle ist;
e) die vorliegende Mischung in einer kontinuierlich betriebenen Umlaufapparatur, enthaltend die Einheit (1) und mindestens eine Destillationseinheit (2) zur Phenol/Wassertrennung, entwässert wird; wobei in der Einheit (1) der wasserfeuchte Ionentauscher, vorzugsweise unter einer Inertgasatmosphäre, mit Phenol bei 50 bis 90°C, bevorzugt 60 bis 80°C, gerührt wird und ein Phenolteilstrom von bis zu 20 % des Gesamtvolumens pro Stunde über eine Leitung (4) ausgespeist und in die Destillationseinheit (2) überführt wird und in der Destillationseinheit (2) eine Auftrennung in Phenol (Sumpfprodukt) und Wasser/Phenol-Mischung (Kopfprodukt) durchgeführt wird und das Phenol über die Leitung (4') in die Einheit (1) zurückgeführt wird. Das phenolhaltige Wasser wird vorzugsweise einer im Herstellungsverfahren vorhandenen Extraktionsapparatur zugeführt. Als Destillationseinheit (2) wird insbesondere eine im Herstellungsverfahren gegebenenfalls integrierte Destillationskolonne zur Wasser/Phenol-Abtrennung genutzt. Das in diesem Teilschritt abgetrennte Kopfprodukt enthält 2 bis 20 Gew.-%, bevorzugt 5 bis 10 % Phenol. Das auf diesem Wege aus dem System ausgetragene Phenol wird durch frisches Phenol in der Einheit (1) ergänzt. Die Mischung wird solange durch die Umlaufapparatur geführt bis ein Restwassergehalt am Ablauf der Einheit (1) von vorzugsweise kleiner 3 Vol%, bevorzugt kleiner 1 Vol% gemessen wird;
f) die Mischung anschließend, vorzugsweise unter inerten Bedingungen, in Form einer Ionenaustauscherharz/Phenol-Suspension in eine geeignetes Reaktionsgefäß überführt wird, wobei die Ionenaustauscherharz/Phenol-Suspension vorzugsweise bei Temperaturen von 50 bis 80°C und vorzugsweise bei einem Feststoffanteil von 40 bis 80 Vol % in pumpfähiger Form vorliegt und vorzugsweise
g) das überstehende Phenol aus dem Reaktionsgefäß abgelassen wird.

Der auf diesem Wege konditionierte Ionentauscher ist hervorragend zur Herstellung von Bisphenolen, insbesondere zur Herstellung von BPA aus Aceton und Phenol geeignet. Das mit diesem Ionentauscher hergestellte BPA weist hohe Produktqualität auf und ist in besonderer Weise zur Herstellung von Polymeren wie Epoxharzen und insbesondere Polycarbonat geeignet.

Das im erfindungsgemäßen Verfahren bei der Konditionierung des Ionenaustauscher unter Punkt g) anfallende Phenol ist mit sauren löslichen Anteilen aus dem Ionenaustauscher kontaminiert. Zur beliebigen Weiterverwendung, insbesondere als Ausgangsstoff zur Herstellung von BPA, wird deshalb das so anfallende Phenol gegebenenfalls vorzugsweise durch Destillation gereinigt, wobei der Sumpf der Kolonne mit bis zu 5 Gew.-% einer basischen Verbindung beschickt wird, die geeignet ist saure Bestandteile zurückzuhalten. Eine bevorzugte basische Verbindungen ist Natriumhydroxid.

Ein Vorteil des beschriebenen Verfahrens besteht darin, daß auf diesem Wege störende saure Oligomeranteile aus dem Ionentauscher entfernt werden (dies führt z.B. zu verbesserter Produktqualität). Durch die Katalysatorvorbehandlung in einem externen Behälter kann während der Konditionierung das Herstellungsverfahren im Reaktionsgefäß fortgeführt werden. Die kontinuierliche Entwässerung des Katalysators mit Phenol-Kreislaufführung erfordert eine Minimalmenge an Phenol, es sind keine zusätzlichen, technisch aufwendigen Apparaturen wie zum Beispiel Vakuum oder Schleppgastrocknung nötig. Durch die Bewegung der Suspension bei der Entwässerung treten keine mechanischen Spannungen durch die Volumenverringerung der Ionentauscherpartikel auf. Entstehende Nebenströme können in einer Produktions-Anlage in denen die Ionenaustauscher als Katalysatoren eingesetzt werden und in der Destillations- und Extraktionseinheiten vorhanden sind aufgearbeitet werden. Dabei kann auch der Großteil des zur Entwässerung verwendeten Phenols in dem Herstellungsverfahren eingesetzt werden.

Das mit einem solchen Katalysatorharz hergestellte BPA weist hohe Produktqualität auf und ist in besonderer Weise zur Herstellung von Polymeren wie Epoxyharzen und insbesondere Polycarbonaten geeignet. Verwendet werden die so hergestellten Polycarbonate zur Herstellung von Formkörpern wie insbesondere Compact Disks, Linsen und Scheiben.

Gegenstand der vorliegenden Anmeldung ist weiterhin ein Verfahren zur Konditionierung von monodispersen Ionenaustauschern zur Katalyse von Kondensationsreaktionen, Additionsreaktionen, Umesterungen oder Alkylierungsreaktionen, dadurch gekennzeichnet, dass man
a) wasserfeuchte monodisperse Ionenaustauscher in sauerstofffreiem VE-Wasser suspendiert,
b) den monodispersen Ionenaustauscher einer diskontinuierlichen oder kontinuierlichen Wäsche mit sauerstoffreiem VE-Wasser bis zur konstanten Leitfähigkeit unterzieht,
c) nach der letzten Wäsche das VE Wasser ablässt und dem monodispersen Ionenaustauscher die sauerstofffreie OH-Komponente zumischt,
d) die erhaltene Mischung in einer kontinuierlich betriebenen Umlaufapparatur mit Destillationseinheit entwässert und
e) schließlich die Suspension aus monodispersem Ionenaustauscher und OH-Komponente in ein Reaktionsgefäß überführt.

Gegenstand der vorliegenden Erfindung sind auch die für die Katalyse der oben genannten Reaktionen konditionierten monodispersen Ionenaustauscher gemäß der Verfahrensschritte a) bis e), sowohl als Kationen- als auch als Anionenaustauscher.

VE-Wasser bedeutet gemäß der vorliegenden Erfindung vollentsalztes Wasser. Als mögliche OH-Komponenten für Kondensationsreaktionen im Sinne der vorliegenden Erfindung kommen beispielsweise Phenole, ortho-, meta-, para-Kresole oder α-, oder β-Naphthole in Frage.

Die bei der Katalyse der oben genannten Reaktionen, insbesondere von Kondensationsreaktionen gemäß der vorliegenden Anmeldung zu konditionierenden monodispersen Ionenaustauscher weisen mikroporöse oder gelförmige oder makroporöse Strukturen auf. Diese Strukturen sind in der Fachliteratur eingehend beschrieben worden. Bevorzugt werden zur Katalyse der oben genannten Reaktionen, insbesondere von Kondensationsreaktionen jedoch makroporöse oder gelförmige monodisperse Ionenaustauscher eingesetzt. Bei der BPA Synthese werden beispielsweise gemäß der vorliegenden Erfindung besonders bevorzugt gelförmige monodisperse Ionenaustauscher eingesetzt.

Die gemäß der vorliegenden Erfindung für die Katalyse der oben genannten Reaktionen, insbesondere von Kondensationsreaktionen zu konditionierenden monodispersen Ionenaustauscher können nach bekannten Verfahren hergestellt werden.

Beispielhaft werden monodisperse Ionenaustauscher gemäß der US-A-4,444,961 oder der DE-A 19 852 667 hergestellt.

Als monodisperse Ionenaustauscher werden in der vorliegenden Anmeldung solche Stoffe bezeichnet, bei denen mindestens 90 Volumen- oder Massen-% der Teilchen einen Durchmesser besitzen, der in dem Intervall mit der Breite von ± 10 % des häufigsten Durchmessers um den häufigsten Durchmesser herum liegt.

Zum Beispiel bei einem Stoff mit häufigstem Durchmesser von 0,5 mm liegen mindestens 90 Volumen- oder Massen-% in einem Größenintervall zwischen 0,45 mm und 0,55 mm, bei einem Stoff mit häufigstem Durchmesser von 0,7 mm liegen mindestens 90 Volumen- oder Massen-% in einem Größenintervall zwischen 0,77 mm und 0,63 mm.

Das erfindungsgemäße Verfahren zur Konditionierung monodisperser Ionenaustauscher vermeidet jedoch nicht nur die oben beschriebenen Nachteile der bekannten Konditionierungsverfahren, sondern ermöglicht gleichzeitig den optimalen Einsatz der konditionierten monodispersen Ionenaustauscher als Katalysatorharze bei Kondensationsreaktionen, bevorzugt bei Kondensationsreaktionen ausgehend von Phenolen, o-, m-, p-Kresolen oder α- oder β-Naphtholen, insbesondere bevorzugt bei der Synthese von Bisphenolen, ganz besonders bevorzugt zur Synthese von BPA aus Phenolen und Aldehyden oder Ketonen.

Die konditionierten monodispersen Ionenaustauscher eignen sich aber auch hervorragend zum Einsatz als Katalysatoren bei Additionsreaktionen. Beispielsweise sei als Additionsreaktion die Addition von Alkoholen an Alkene genannt, bevorzugt von Alkoholen an C₁-C₄-Alkene, insbesondere bevorzugt von Methanol, Ethanol, Propanol oder Butanol an Isobuten, ganz besonders bevorzugt von Methanol an Isobuten zu Methyl-t-butylether.

Die konditionierten monodispersen Ionenaustauscher eignen sich zudem zur Katalyse von Veresterungen durch Reaktion von Alkoholen mit Carbonsäuren, bevorzugt von C₁-C₈-Alkoholen mit C₁-C₈-Carbonsäuren, besonders bevorzugt zur Veresterung von Methanol, Ethanol, Propanol und allen Isomeren des Butanols mit Carbonsäuren der Reihe Ameisensäure, Essigsäure, Propionsäure oder Buttersäure.

Außerdem eignen sich die konditionierten monodispersen Ionenaustauscher zur Katalyse von Umesterungsreaktionen, beispielsweise von Triestem zu Monoestern, insbesondere die Umesterung eines Triglycerids mit Methanol, Ethanol, Propanol oder Butanol zu einem Fettsäuremonoester.

Schließlich eignen sich die konditionierten monodispersen Ionenaustauscher zur Katalyse von Alkylierungsreaktionen, beispielsweise die Alkylierung von Phenolen oder Kresolen mit linearen oder verzweigten Olefinen beispielsweise zu Triisobuten oder Nonen.

Bevorzugter Gegenstand der vorliegenden Anmeldung ist ein Verfahren zur Konditionierung von monodispersen Ionenaustauschern zur Katalyse von Kondensationsreaktionen, insbesondere der Synthese von Bisphenolen, dadurch gekennzeichnet, dass
a) der wasserfeuchte monodisperse Ionenaustauscher in einer Einheit (1) mit sauerstoffreiem VE-Wasser bei 5 bis 80°C, insbesondere 20 bis 60°C suspendiert wird; wobei insbesondere ein Volumenverhältnis von 3 bis 1,5 Teilen, bevorzugt 2,5 bis 2 Teilen wasserfeuchtem Ionenaustauscher zu 1 Teil Wasser eingestellt wird und wobei der Gehalt an gelöstem Sauerstoff im eingesetzten VE-Wasser vorzugsweise nicht grösser als 1 ppm, bevorzugt nicht grösser als 100 ppb, ist, und wobei der Gehalt an gelösten oder ungelösten Metallionen im verwendeten VE-Wasser vorzugsweise nicht grösser als 1 ppm, bevorzugt nicht grösser als 0,5 ppm für Fe, Co, Ni, Mo, Cr, Cu als individuelle Komponenten ist und vorzugsweise nicht größer als 10 ppm, bevorzugt nicht größer als 1 ppm für die Summe der genannten Metalle ist;
b) die Suspension, vorzugsweise durch ½ bis 24-stündiges Rühren bei 5 bis 80°C, insbesondere 20 bis 60°C bewegt wird und anschließend eine Analyse der überstehenden wässrigen Lösung auf ihre Leitfähigkeit durchgeführt wird, um einen Anhaltspunkt über den Oligomerengehalt des monodispersen Ionenaustauschers und die Anzahl der nötigen Waschzyklen für Schritt c) zu erhalten;
c) der monodisperse Ionenaustauscher einer diskontinuierlichen Wäsche mit sauerstofffreiem VE-Wasser bis zur konstanten Restleitfähigkeit unterzogen wird, in Abhängigkeit der Leitfähigkeit wird vorzugsweise 5 bis 50, insbesondere 10 bis 25 mal gewaschen; wobei nach Ablassen des VE-Wassers jeweils 0,2 bis 1,5 Volumenteile VE-Wasser, bezogen auf den wasserfeuchten monodispersen Ionenaustauscher hinzugefügt, die Mischung bewegt und das VE Wasser abgelassen und am Ablauf die Leitfähigkeit überprüft wird, wobei die Leitfähigkeit am Ende der Waschzyklen kleiner 100 mikro-Siemens/cm, bevorzugt kleiner 50 mikro-Siemens/cm, besonders bevorzugt kleiner 20 mikro-Siemens/cm ist und sich mit einer Abweichung von kleiner 20 mikro-Siemens/cm von Wäsche zu Wäsche konstant verhält, und wobei der Gehalt an gelöstem Sauerstoff im eingesetzten VE-Wasser vorzugsweise nicht grösser als 1 ppm, bevorzugt nicht grösser als 100 ppb, ist und wobei der Gehalt an gelösten oder ungelösten Metallionen im verwendeten VE-Wasser vorzugsweise nicht größer als 1 ppm, bevorzugt nicht größer als 0,5 ppm für Fe, Co, Ni, Mo, Cr, Cu als individuelle Komponenten ist und vorzugsweise nicht größer als 10 ppm, bevorzugt nicht grösser als 1 ppm für die Summe der genannten Metalle ist;
d) nach der letzten Wäsche das VE Wasser abgelassen und dem monodispersen Ionenaustauscher sauerstofffreies Phenol bei 50 bis 90°C, insbesondere 60 bis 80°C zugemischt wird; wobei vorzugsweise ein Volumenverhältnis von 0,60 bis 1,5 Teilen von wasserfeuchtem monodispersen Ionenaustauscher zu einem Teil Phenol eingestellt wird und wobei vorzugsweise der Gehalt an gelöstem Sauerstoff des verwendeten Phenols kleiner 1 ppm, bevorzugt kleiner 100 ppb ist, und wobei der Gehalt an gelösten oder ungelösten Metallionen im verwendeten Phenol vorzugsweise nicht grösser als 1 ppm, bevorzugt nicht grösser als 0,5 ppm für Fe, Co, Ni, Mo, Cr, Cu als individuelle Komponenten ist und vorzugsweise nicht größer als 10 ppm, bevorzugt nicht grösser als 1 ppm für die Summe der genannten Metalle ist;
e) die vorliegende Mischung in einer kontinuierlich betriebenen Umlaufapparatur, enthaltend die Einheit (1) und mindestens eine Destillationseinheit (2) zur Phenol/Wassertrennung, entwässert wird; wobei in der Einheit (1) der wasserfeuchte Ionenaustauscher, vorzugsweise unter einer Inertgasatmosphäre, mit Phenol bei 50 bis 90°C, bevorzugt 60 bis 80°C, gerührt wird und ein Phenolteilstrom von bis zu 20 % des Gesamtvolumens pro Stunde über eine Leitung (4) ausgespeist und in die Destillationseinheit (2) überführt wird und in der Destillationseinheit (2) eine Auftrennung in Phenol (Sumpfprodukt) und Wasser/Phenol-Mischung (Kopfprodukt) durchgeführt wird und das Phenol über die Leitung (4') in die Einheit (1) zurückgeführt wird. Das phenolhaltige Wasser wird vorzugsweise einer im Herstellungsverfahren vorhandenen Extraktionsapparatur zugeführt. Als Destillationseinheit (2) wird insbesondere eine im Herstellungsverfahren gegebenenfalls integrierte Destillationskolonne zur Wasser/Phenol-Abtrennung genutzt. Das in diesem Teilschritt abgetrennte Kopfprodukt enthält 2 bis 20 Gew.-%, bevorzugt 5 bis 10 Gew.-% Phenol. Das auf diesem Wege aus dem System ausgetragene Phenol wird durch frisches Phenol in der Einheit (1) ergänzt. Die Mischung wird solange durch die Umlaufapparatur geführt, bis ein Restwassergehalt am Ablauf der Einheit (1) von vorzugsweise kleiner 3 Vol%, bevorzugt kleiner 1 Vol% gemessen wird;
f) die Mischung anschließend, vorzugsweise unter inerten Bedingungen, in Form einer Ionenaustauscherharz/Phenol-Suspension in ein geeignetes Reaktionsgefäß überführt wird, wobei die Ionenaustauscherharz/Phenol-Suspension vorzugsweise bei Temperaturen von 50 bis 80°C und vorzugsweise bei einem Feststoffanteil von 40 bis 80 Vol % in pumpfähiger Form vorliegt und vorzugsweise
g) das überstehende Phenol aus dem Reaktionsgefäß abgelassen wird.

Weiterhin bevorzugter Gegenstand der vorliegenden Erfindung sind die zur Synthese von Bisphenolen konditionierten monodispersen Ionenaustauscher gemäß der Verfahrensschritte a) bis g), in der Kationform.

Die auf den oben beschriebenen Wegen konditionierten monodispersen Ionentauscher sind hervorragend zur Durchführung von Kondensationsreaktionen, beispielsweise zur Herstellung von Bisphenolen, insbesondere zur Herstellung von BPA aus Aceton und Phenol geeignet. Das mit diesem konditionierten monodispersen Ionentauscher hergestellte BPA weist hohe Produktqualität auf und ist in besonderer Weise zur Herstellung von Polymeren wie Epoxharzen und insbesondere Polycarbonat geeignet.

Das im erfindungsgemäßen Verfahren bei der Konditionierung des monodispersen Ionenaustauschers im Falle der Synthese von Bisphenolen unter Punkt g) anfallende Phenol ist mit sauren löslichen Anteilen aus dem Ionenaustauscher kontaminiert. Zur beliebigen Weiterverwendung, insbesondere als Ausgangsstoff zur Herstellung von BPA, wird deshalb das so anfallende Phenol gegebenenfalls vorzugsweise durch Destillation gereinigt, wobei der Sumpf der Kolonne mit bis zu 5 Gew.-% einer basischen Verbindung beschickt wird, die geeignet ist, saure Bestandteile zurückzuhalten. Eine bevorzugte basische Verbindung ist Natriumhydroxid.

Ein Vorteil des zur Herstellung von Bisphenolen, insbesondere bevorzugt zur Herstellung von BPA beschriebenen Verfahrens besteht darin, dass auf diesem Wege störende saure Oligomeranteile aus dem monodispersen Ionentauscher entfernt werden (dies führt z.B. zu verbesserter Produktqualität). Durch die Katalysatorvorbehandlung in einem externen Behälter kann während der Konditionierung das Herstellungsverfahren im Reaktionsgefäß fortgeführt werden. Die kontinuierliche Entwässerung des Katalysators mit Phenol-Kreislaufführung erfordert eine Minimalmenge an Phenol, es sind keine zusätzlichen, technisch aufwendigen Apparaturen wie zum Beispiel Vakuum oder Schleppgastrocknung nötig. Durch die Bewegung der Suspension bei der Entwässerung treten keine mechanischen Spannungen durch die Volumenverringerung der Ionentauscherpartikel auf. Entstehende Nebenströme können in einer Produktions-Anlage in denen die monodispersen Ionenaustauscher als Katalysatoren eingesetzt werden und in der Destillations- und Extraktionseinheiten vorhanden sind, aufgearbeitet werden. Dabei kann auch der Großteil des zur Entwässerung verwendeten Phenols in dem Herstellungsverfahren eingesetzt werden.

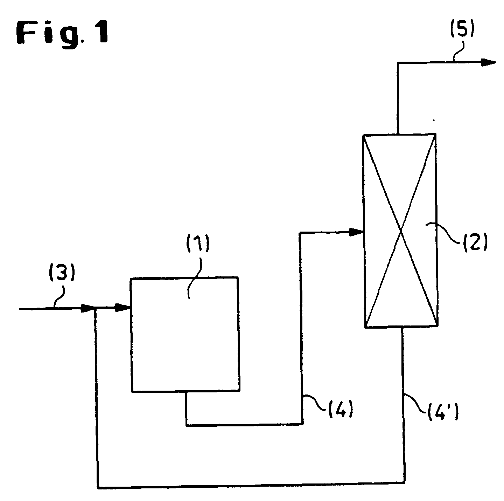

In Abb.1 steht
(1) für einen Rührbehälter
(2) für eine Destillationseinheit
(3) für eine Phenolzuleitung
(4) für Phenolumlaufleitungen
(5) für Wasser/(Phenol)austragsleitung

Darüber hinaus und dies ist ebenfalls Gegenstand der vorliegenden Erfindung können die gemäß dem in dieser Anmeldung beanspruchten Konditionierungsverfahren hergestellten konditionierten monodispersen Ionenaustauscher für eine Vielzahl weiterer Reaktionen als Katalysatoren eingesetzt werden.

So eignen sich die konditionierten monodispersen Ionenaustauscher in der Kationoder Anion-Form als Katalysatoren nicht nur für Kondensationsreaktionen sondern auch für Additionsreaktionen, beispielsweise von Alkoholen an Alkenen zu Ethern, als Katalysatoren bei Veresterungen oder Umesterungen aber auch als Katalysatoren bei Alkylierungsreaktionen.

Beispielsweise kann mit dem erfindungsgemäß konditionierten Ionenaustauschern in der Kation-Form die Umsetzung von Methanol mit Isobuten oder Isopenten zu Methyl-t-butyl-ether oder t-Amyl-methylether katalysiert werden.

Monodisperse Ionenaustauscher sind erfindungsgemäß bevorzugt.

Die erfindungsgemäß konditionierten Ionenaustauscher sind besonders geeignet für die Herstellung von Bisphenolen, insbesondere von BPA.

Die nachfolgenden Beispiele dienen zur Erläuterung der Erfindung. Die Erfindung ist nicht auf die Beispiele limitiert.

### Beispiele

### Beispiel 1

Kommerziell erhältlicher Ionentauscher (Lewatit SC 104, Bayer AG) wird nach oben beschriebenem Schema präpariert. Die Wasserwäsche (Sauerstoffgehalt im VE-Wasser 50 ppb) erfolgt unter Stickstoffatmosphäre in 12 Zyklen bei 30°C, die Restleitfähigkeit des Waschwassers am Ablauf beträgt beim letzten Zyklus 14 mikro-Siemens/cm. Die Entwässerung erfolgt kontinuierlich unter Stickstoffatmosphäre bei 70°C mit 0,60 Volumenteilen Phenol (Sauerstoffgehalt 50 ppb). Hierbei verringert sich das Volumen des Ionentauschers um 40 %. Das am Ablauf anfallende wasserhaltige Phenol wird durch Destillation bei anfänglich 700 mbar und 105°C Sumpftemperatur abdestlliert. Zum Ende der Destillation wird das Vakuum auf 130 mbar abgesenkt die Sumpftemperatur steigt auf 125°C. Auf diese Weise wird pro Stunde in ansteigender Menge 1 bis 10 % der gesamt vorhandenen Flüssigkeitsmenge im Rührbehälter über die Destillationskolonne geführt. Phenol wird als Sumpfprodukt in den Entwässerungsbehälter zurückgeführt. Das Kopfprodukt der Kolonne (8 % Phenol, 92 % Wasser) wird einer kontinuierlichen Extraktionsanlage zugeführt. Auf diese Weise ausgeschleustes Phenol wird kontinuierlich durch Frischphenol ergänzt.

Die kontinuierliche Entwässerung wird beendet, wenn am Ablauf ein Wassergehalt von 0,2 % Wasser in Phenol erreicht wird. Der Ionentauscher wird bei 70°C als Suspension in Phenol (Feststoffanteil 40 Vol-%) in das Reaktionsgefäß überführt. Überstehendes Phenol wird abgelassen und durch Destillation bei 120°C, 150 mbar über Natriumhydroxyd (0,001 Gew.-%) gereinigt.

Der so präparierte Ionentauscher wird zur kontinuierlichen Produktion von 2,2-Bis(4-hydroxyphenyl)propan aus einer Mischung von Phenol (96 %) und Aceton (4 %) bei 65°C genutzt. Hierbei wird bei einem Durchsatz von 0,15 l/l * h ein Acetonumsatz von 96 % und eine Selektivität von 93,5 % BPA erzielt. Die Farbzahl am Ablauf des Reaktors beträgt 5 Hazen. Die Reaktionsmischung am Ablauf des Reaktors wird anschließend 4 h bei 190°C getempert, hierbei erhöhte sich die Farbzahl von 5 Hazen auf 15 Hazen.

Wird die Reaktion mit dem durch alkalische Destillation zurückgewonnenen Phenol durchgeführt, kann keine meßbaren Unterschiede bezüglich Umsatz, Selektivität, Farbzahl und Farbentwicklung bei Temperung festgestellt werden.

### Vergleichsbeispiel 2

Die Ionentauscherpräparation wird in der in Bsp. 1 beschriebenen Weise durchgeführt, jedoch wird das zur Entwässerung genutzte Phenol anschließend direkt ohne alkalische Destillation zur BPA-Herstelllung genutzt. Der Acetonumsatz betrug 94 %, die Selektivität 93,0 % und die Hazen-Farbzahl am Ablauf des Reaktors 10 Hazen. Dieser Wert steigt nach Temperung (4 h bei 190°C) auf 30 Hazen an.

### Vergleichsbeispiel 3

Kommerziell erhältlicher Ionentauscher (Lewatit SC 104, Bayer AG) wird analog zu Beispiel 1 präpariert und zur Herstellung von BPA genutzt, jedoch wird auf eine anfängliche Wasserwäsche verzichtet. Es wird ein Acetonumsatz von 94 % und eine Selektivität von 92,5 % beobachtet. Die Hazen-Farbzahl der Reaktionsmischung beträgt 15 Hazen. Der Wert steigt nach 4 h bei 190°C auf 40 Hazen an.

### Vergleichsbeispiel 4

Kommerziell erhältlicher Ionentauscher (Lewatit SC 104, Bayer AG) wird analog zu Beispiel 1 präpariert und zur Herstellung von BPA genutzt, jedoch wird bei der Entwässerung mit Phenol am Ende ein Wasserwert von 10 % im ablaufenden Phenol erreicht. Es wird ein Acetonumsatz von 86 % und eine Selektivität von 93,0 % beobachtet. Die Hazen-Farbzahl der Reaktionsmischung beträgt 10 Hazen. Der Wert steigt nach Temperung (4 h bei 190°C) auf 25 Hazen an.

### Vergleichsbeispiel 5

Kommerziell erhältlicher Ionentauscher (Lewatit SC 104, Bayer AG) wurde analog zu Beispiel 1 präpariert und zur Herstellung von BPA genutzt, jedoch wurde auf eine intensive Inertisierung bei der Wasserwäsche verzichtet. Außerdem enthielt das zur Wäsche verwendete VE-Wasser 2 ppm gelösten Sauerstoff, 5 ppm Fe, 2 ppm Co und 2 ppm Ni. Es wurde ein Acetonumsatz von 93 % und eine Selektivität von 93,0 % beobachtet. Die Hazen-Farbzahl der Reaktionsmischung betrug 15 Hazen. Der Wert steig nach Temperung (4 h bei 190°C) auf 30 Hazen an.

### Beispiel 6

### Kondensationsreaktion am Beispiel der Synthese von Bisphenolen, hier BPA:

Kommerziell erhältlicher monodisperser Ionentauscher (Lewatit K 1261®, Bayer AG) wird nach oben beschriebenem Schema präpariert. Die Wasserwäsche (Sauerstoffgehalt im VE-Wasser 50 ppb) erfolgt unter Stickstoffatmosphäre in 12 Zyklen bei 30°C, die Restleitfähigkeit des Waschwassers am Ablauf beträgt beim letzten Zyklus 14 mikro-Siemens/cm. Die Entwässerung erfolgt kontinuierlich unter Stickstoffatmosphäre bei 70°C mit 0,60 Volumenteilen Phenol (Sauerstoffgehalt 50 ppb). Hierbei verringert sich das Volumen des monodispersen Ionentauschers um 40 %. Das am Ablauf anfallende wasserhaltige Phenol wird durch Destillation bei anfänglich 700 mbar und 105°C Sumpftemperatur abdestilliert. Zum Ende der Destillation wird das Vakuum auf 130 mbar abgesenkt, die Sumpftemperatur steigt auf 125°C. Auf diese Weise wird pro Stunde in ansteigender Menge 1 bis 10 % der gesamt vorhandenen Flüssigkeitsmenge im Rührbehälter über die Destillationskolonne geführt. Phenol wird als Sumpfprodukt in den Entwässerungsbehälter zurückgeführt. Das Kopfprodukt der Kolonne (8 % Phenol, 92 % Wasser) wird einer kontinuierlichen Extraktionsanlage zugeführt. Auf diese Weise ausgeschleustes Phenol wird kontinuierlich durch Frischphenol ergänzt.

Die kontinuierliche Entwässerung wird beendet, wenn am Ablauf ein Wassergehalt von 0,2 % Wasser in Phenol erreicht wird. Der monodisperse Ionentauscher wird bei 70°C als Suspension in Phenol (Feststoffanteil 40 Vol-%) in das Reaktionsgefäß überführt. Überstehendes Phenol wird abgelassen und durch Destillation bei 120°C, 150 mbar über Natriumhydroxyd (0,001 Gew.-%) gereinigt.

Der so präparierte monodisperse Ionentauscher wird zur kontinuierlichen Produktion von 2,2-Bis(4-hydroxyphenyl)propan aus einer Mischung von Phenol (96 %) und Aceton (4 %) bei 65°C genutzt. Hierbei wird bei einem Durchsatz von 0,15 l/l • h ein Acetonumsatz von 96 % und eine Selektivität von 93,5 % BPA erzielt. Die Farbzahl am Ablauf des Reaktors beträgt 5 Hazen. Die Reaktionsmischung am Ablauf des Reaktors wird anschließend 4 h bei 190°C getempert, hierbei erhöhte sich die Farbzahl von 5 Hazen auf 15 Hazen.

Wird die Reaktion mit dem durch alkalische Destillation zurückgewonnenen Phenol durchgeführt, kann kein messbarer Unterschied bezüglich Umsatz, Selektivität, Farbzahl und Farbentwicklung bei Temperung festgestellt werden.

### Vergleichsbeispiel 7

Kommerziell erhältlicher heterodisperser Ionentauscher (Lewatit SC 104®, Bayer AG) wird analog zu Beispiel 1 präpariert und zur Herstellung von BPA genutzt, jedoch wird auf eine anfängliche Wasserwäsche verzichtet. Es wird ein Acetonumsatz von 94 % und eine Selektivität von 92,5 % beobachtet. Die Hazen-Farbzahl der Reaktionsmischung beträgt 15 Hazen. Der Wert steigt nach 4 h bei 190°C auf 40 Hazen an.

## Patentansprüche

1. Verfahren zur Konditionierung von monodispersen Ionenaustauschern zur Katalyse von Kondensationsreaktionen, Additionsreaktionen, Umesterungen, Veresterungen oder Alkylierungsreaktionen, **dadurch gekennzeichnet, dass** man
a) wasserfeuchte monodisperse Ionenaustauscher in sauerstofffreiem VE-Wasser suspendiert,
b) den monodispersen Ionenaustauscher einer diskontinuierlichen oder kontinuierlichen Wäsche mit sauerstoffreiem VE-Wasser bis zur konstanten Leitfähigkeit unterzieht,
c) nach der letzten Wäsche das VE Wasser ablässt und dem monodispersen Ionenaustauscher die sauerstofffreie OH-Komponente zumischt,
d) die erhaltene Mischung in einer kontinuierlich betriebenen Umlaufapparatur mit Destillationseinheit entwässert und
e) schließlich die Suspension aus monodispersem Ionenaustauscher und OH-Komponente in ein Reaktionsgefäß überführt, wobei eingesetzte sauerstofffreie VE-Wasser einen Gehalt an gelösten Sauerstoff von nicht größer als 1ppm und einen Gehalt an gelösten oder ungelösten Metallionen von nicht größer als 1ppm für Fe, Co, Ni, Mo, Cr, Cu, als individuelle Komponenten aufweist.
wobei eingesetzte sauerstofffreie VE-Wasser einen Gehalt an gelösten Sauerstoff von nicht größer als 1ppm und einen Gehalt an gelösten oder ungelösten Metallionen von nicht größer als 1ppm für Fe, Co, Ni, Mo, Cr, Cu, als individuelle Komponenten aufweist.

2. Verfahren zur Konditionierung von Ionenaustauschern, **dadurch gekennzeichnet, dass**
a) der wasserfeuchte Ionentauscher in einer Einheit (1) mit sauerstoffreiem VE-Wasser bei 5 bis 80°C suspendiert wird;
b) die Suspension bewegt und anschließend eine Analyse der überstehenden wässrigen Lösung auf Leitfähigkeit durchgeführt wird;
c) der Ionenaustauscher einer diskontinuierliche Wäsche mit sauerstofffreiem VE-Wasser bis zu konstanter Restleitfähigkeit unterzogen wird) wobei die Leitfähigkeit am Ende der Waschzyklen kleiner als 100 µSiemens/cm ist;
d) nach der letzten Wäsche das VE-Wasser abgelassen und dem Ionenaustauscher sauerstofffreies Phenol bei Temperaturen von 50 bis 90°C zugemischt wird;
e) die erhaltene Mischung in einer kontinuierlich betriebenen Umlaufapparatur, enthaltend die Einheit (1) und mindestens eine Destillationseinheit (2) zur Phenol/Wassertrennung, entwässert wird;
f) die Mischung anschließend in Form einer Ionenaustauscher/Phenol-Suspension in ein Reaktkionsgefäß überführt wird, wobei eingesetzte sauerstofffreie VE-Wasser einen Gehalt an gelösten Sauerstoff von nicht größer als 1ppm und einen Gehalt an gelösten oder ungelösten Metallionen von nicht größer als 1ppm für Fe, Co, Ni, Mo, Cr, Cu, als individuelle Komponenten aufweist.

3. Verfahren nach Anspruch 2, wobei der Ionenaustauscher monodispers ist.

4. Verfahren zur Konditionierung von monodispersen Ionentauschern gemäß Anspruch 3, **dadurch gekennzeichnet, dass** nach der Überführung der Ionentauscher/Phenol-Suspension in das Reaktionsgefäß überstehendes Phenol abgelassen und durch Destillation über basische Verbindungen aufgereinigt wird.

5. Verfahren gemäß Anspruch 1, **dadurch gekennzeichnet, dass** die monodispersen Ionenaustauscher eine gelförmige oder makroporöse Struktur aufweisen.

6. Verfahren gemäß Anspruch 2, wobei der Gehalt an gelösten oder ungelösten Metallionen im verwendeten VE-Wasser, nicht größer als 0,5 ppm für Fe, Co, Ni, Mo, Cr, Cu als individuelle Komponenten ist und vorzugsweise nicht größer als 10 ppm, bevorzugt nicht größer als 1 ppm für die Summe der genannten Metalle ist.

7. Verfahren zur Konditionierung von Ionentauschem gemäß Anspruch 2, **dadurch gekennzeichnet, dass** nach der Überführung der Ionentauscher/Phenol-Suspension in das Reaktionsgefäß überstehendes Phenol abgelassen und durch Destillation über basische Verbindungen aufgereinigt wird.

8. Konditionierte Ionenaustauscher erhältlich nach dem Verfahren gemäß einem der Ansprüche 1 bis 7.

9. Konditionierte Ionenaustauscher gemäß Anspruch 8, wobei diese in der Kationoder Anion-Form vorliegen.

10. Verwendung des Ionenaustauschers nach Anspruch 8 als Katalysator.

11. Verwendung des Ionenaustauschers nach Anspruch 8 als Katalysator für die Herstellung von Bisphenolen.

12. Verwendung des Ionenaustauschers nach Anspruch 1 als Katalysatoren bei Kondensationsreaktionen, Additionsreaktionen, Umesterungen, Veresterungen oder Alkylierungsreaktionen, wobei der Ionenaustauscher monodispers ist.

13. Verwendung gemäß Anspruch 12, **dadurch gekennzeichnet, dass** die monodispersen Ionenaustauscher bei Kondensationsreaktionen ausgehend von Phenolen, ortho-, meta-, para-Kresolen oder α- oder β-Naphtholen eingesetzt werden.

## Claims

1. A process for conditioning monodisperse ion-exchangers for the catalysis of condensation reactions, addition reactions, transesterifications, esterifications or alkylation reactions, **characterised in that**
a) monodisperse ion-exchangers which have been moistened with water are suspended in oxygen-free, completely de-ionized water,
b) the monodisperse ion-exchanger is subjected to discontinuous or continuous washing with oxygen-free, completely de-ibnized water until constant conductivity is attained,
c) after the last wash the completely de-ionized water is discharged and the oxygen-free OH component is admixed to the monodisperse ion-exchanger,
d) the mixture which is obtained is dehydrated in a continuously operated circulating apparatus with distillation unit and
e) finally the suspension consisting of monodisperse ion-exchanger and OH component is transferred into a reaction vessel, the oxygen-free, completely de-ionized water which is employed having a content of dissolved oxygen of no greater than 1 ppm and a content of dissolved or undissolved metal ions of no greater than 1 ppm for Fe, Co, Ni, Mo, Cr, Cu as individual components.

2. A process for conditioning ion-exchangers,
**characterised in that**
a) the ion-exchanger which has been moistened with water is suspended in a unit (1) with oxygen-free, completely de-ionized water at 5 to 80°C;
b) the suspension is agitated and subsequently an analysis of the supernatant aqueous solution in respect of conductivity is carried out;
c) the ion-exchanger is subjected to discontinuous washing with oxygen-free, completely de-ionized water until constant residual conductivity is attained, the conductivity at the end of the washing cycle being less than 100 µSiemens/cm;
d) after the last wash the completely de-ionized water is discharged, and oxygen-free phenol is admixed to the ion-exchanger at temperatures from 50 to 90°C;
e) the mixture that is obtained is dehydrated in a continuously operated circulating apparatus containing the unit (1) and at least one distillation unit (2) for the purpose of separating phenol and water;
f) the mixture.is subsequently transferred in the form of an ion-exchanger/phenol suspension into a reaction vessel, the oxygen-free, completely de-ionized water which is employed having a content of dissolved oxygen of no greater than 1 ppm and a content of dissolved or undissolved metal ions of no greater than 1 ppm for Fe, Co, Ni, Mo, Cr, Cu as individual components.

3. Process according to Claim 2, wherein the ion-exchanger is monodisperse.

4. Process for conditioning monodisperse ion-exchangers according to Claim 3, **characterised in that** after the ion-exchanger/phenol suspension has been transferred into the reaction vessel supernatant phenol is discharged and the degree of purity is increased by distillation over basic compounds.

5. Process according to Claim 1, **characterised in that** the monodisperse ion-exchangers exhibit a gel-like or macroporous structure.

6. Process according to Claim 2, wherein the content of dissolved or undissolved metallic ions in the completely de-ionized water which is used is no greater than 0.5 ppm for Fe, Co, Ni, Mo, Cr, Cu as individual components and is preferably no greater than 10 ppm, favourably no greater than 1 ppm, for the sum of the stated metals.

7. Process for conditioning ion-exchangers according to Claim 2, **characterised in that** after the ion-exchanger/phenol suspension has been transferred into the reaction vessel supernatant phenol is discharged and the degree of purity is increased by distillation over basic compounds.

8. Conditioned ion-exchangers obtainable by the process according to one of Claims 1 to 7.

9. Conditioned ion-exchangers according to Claim 8, wherein said conditioned ion-exchangers are present in the cation form or anion form.

10. Use of the ion-exchanger according to Claim 8 as a catalyst.

11. Use of the ion-exchanger according to Claim 8 as a catalyst for the production of bisphenols.

12. Use of the ion-exchanger according to Claim 1 as catalysts in condensation reactions, addition reactions, transesterifications, esterifications or alkylation reactions, wherein the ion-exchanger is monodisperse.

13. Use according to Claim 12, **characterised in that** the monodisperse ion-exchangers are employed in condensation reactions starting from phenols, ortho-, meta-, para-cresols or α- or β-naphthols.

## Revendications

1. Procédé de conditionnement de résines échangeuses d'ions monodispersées pour la catalyse de réactions de condensation, de réactions d'addition, de transestérifications, d'estérifications ou de réactions d'alkylation, **caractérisé en ce que** :
a) on met en suspension des résines échangeuses d'ions monodispersées humides dans de l'eau ED (entièrement déminéralisée) exempte d'oxygène,
b) on soumet la résine échangeuse d'ions monodispersée à un lavage continu ou discontinu avec de l'eau ED exempte d'oxygène jusqu'à une conductibilité constante,
c) on évacue l'eau ED après le dernier lavage et on ajoute à la résine échangeuse d'ions monodispersée le composant OH exempt d'oxygène,
d) on déshydrate le mélange obtenu dans un appareillage à circulation fonctionnant en continu avec une unité de distillation et
e) finalement, la suspension de la résine échangeuse d'ions monodispersée et du composant OH est amenée dans un récipient réacteur, l'eau ED exempte d'oxygène utilisée présentant un taux d'oxygène dissous n'excédant pas 1 ppm et un taux d'ions métalliques dissous ou non dissous n'excédant pas 1 ppm pour Fe, Co, Ni, Mo, Cr, Cu comme composants individuels.

2. Procédé de conditionnement de résines échangeuses d'ions, **caractérisé en ce que** :
a) on met en suspension la résine échangeuse d'ions humide dans une unité (1) avec de l'eau ED (entièrement déminéralisée) exempte d'oxygène entre 5 et 80°C,
b) la suspension est agitée et, ensuite, une analyse de la solution aqueuse supérieure est effectuée quant à sa conductibilité;
c) la résine échangeuse d'ions est soumise à un lavage discontinu avec de l'eau ED exempte d'oxygène jusqu'à une conductibilité résiduelle constante, la conductibilité à la fin des cycles de lavage étant inférieure à 100 µSiemens/cm;
d) après le dernier lavage, l'eau ED est évacuée et du phénol exempt d'oxygène est ajouté à la résine échangeuse d'ions à des températures de 50 à 90°C;
e) le mélange obtenu est déshydraté dans un appareillage à circulation fonctionnant en continu contenant l'unité (1) et au moins une unité de distillation (2) pour la séparation phénol/eau;
f) le mélange est ensuite transféré dans un récipient réacteur sous la forme d'une suspension résine échangeuse d'ions/phénol, l'eau ED exempte d'oxygène utilisée présentant un taux d'oxygène dissous n'excédant pas 1 ppm et un taux d'ions métalliques dissous ou non dissous n'excédant pas 1 ppm pour Fe, Co, Ni, Mo, Cr, Cu comme composants individuels.

3. Procédé selon la revendication 2, la résine échangeuse d'ions étant monodispersée.

4. Procédé de conditionnement de résines échangeuses d'ions monodispersées selon la revendication 3, **caractérisé en ce que** le phénol surnageant après le transfert de la suspension de la résine échangeuse d'ions/phénol dans le récipient réacteur est évacué et purifié par distillation sur des composés basiques.

5. Procédé selon la revendication 1, **caractérisé en ce que** les résines échangeuses d'ions monodispersées présentent une structure en forme de gel ou macroporeuse.

6. Procédé selon la revendication 2, le taux d'ions métalliques dissous ou non dissous dans l'eau ED utilisée n'étant pas supérieur à 0,5 ppm pour Fe, Co, Ni, Mo, Cr, Cu comme composants individuels et de préférence pas supérieur à 10 ppm, en particulier à 1 ppm pour la somme desdits métaux.

7. Procédé de conditionnement de résines échangeuses d'ions selon la revendication 2, **caractérisé en ce que** le phénol surnageant après le transfert de la suspension résine échangeuse d'ions/phénol dans le récipient réacteur est évacué et purifié par distillation sur des composés basiques.

8. Résine échangeuse d'ions conditionnée obtenue selon le procédé conformément à l'une des revendications 1 à 7.

9. Résine échangeuse d'ions conditionnée selon la revendication 8, celle-ci étant présente sous forme cationique ou anionique.

10. Mise en oeuvre de la résine échangeuse d'ions selon la revendication 8 comme catalyseur.

11. Mise en oeuvre de la résine échangeuse d'ions selon la revendication 8 comme catalyseur pour la préparation de bisphénols.

12. Mise en oeuvre de la résine échangeuse d'ions selon la revendication 1 comme catalyseur pour des réactions de condensation, réactions d'addition, estérifications, transestérifications ou réactions d'alkylation, la résine échangeuse d'ions étant monodispersée.

13. Mise en oeuvre selon la revendication 12, **caractérisée en ce que** les résines échangeuses d'ions monodispersées sont utilisées lors des réactions de condensation à partir de phénols, d'ortho-, méta-, paracrésols ou d'α- ou β-naphtols.
